# DEMANDE DE BREVET EUROPEEN

(11) **EP 2 047 868 A2**
(43) Date de publication de la demande: **15.04.2009**
(21) Numéro de dépôt: 08165937.7
(22) Date de dépôt: 06.10.2008
(51) Int. Cl.: A61L 9/12, B60H 3/00

(54) **Diffuseur d'aérosols à recharges interchangeables et recharges adaptées pour être utilisées dans ce diffuseur.**

(30) Priorité: 12.10.2007 FR 0758262
(71) Demandeur: Jean, Marcel, 06250 Mougins (FR)
(72) Inventeur: Jean, Marcel, 06250 Mougins (FR)
(74) Mandataire: Roman, Alexis

(57) **Abrégé**

La présente invention a pour objet un diffuseur d'aérosols comportant un support (1) destiné à être monté sur un aérateur d'un système de ventilation et des recharges interchangeables,
se caractérisant par le fait que ledit support est formé d'une embase (4) équipée d'un logement (40) en forme de berceau-écrin polyvalent, ledit logement étant configuré pour recevoir successivement :
- le flacon d'une recharge à produit liquide contenant un concentré de parfum liquide, ledit flacon étant surmonté d'un bouchon diffuseur en bois,
- le récipient d'une recharge à produit solide contenant des granulés parfumés, ledit récipient étant surmonté d'un diffuseur,

et par le fait que le logement (40) est surmonté d'un déflecteur d'air (10) disposé en vis-à-vis du bouchon diffuseur en bois de la recharge à produit liquide ou du diffuseur de la recharge à produit solide.

## Description

### Domaine technique de l'invention.

La présente invention a pour objet un diffuseur d'aérosols constitué d'un support monté sur un aérateur de système de ventilation et pouvant recevoir plusieurs systèmes de recharges tels que flacon de verre avec bouchon diffuseur en bois ou container a plusieurs compartiments recevant un parfum solide.

Elle se rapporte d'une manière générale au domaine industriel et commercial des articles destinés à répandre dans l'atmosphère un parfum ou tout autre substance volatile destinée à améliorer le confort de l'environnement, dans tous les lieux comportant un système d'aération ou de climatisation, que ce soit dans un local ou dans un véhicule, le dispositif pouvant également être associé à tout appareil bureautique, domestique ou autre disposant d'un système de ventilation.

### État de la technique.

Il existe à ce jour un grand nombre de dispositifs permettant de diffuser des parfums ou des déodorants, en particulier pour une utilisation dans les automobiles, par exemple en se fixant sur un aérateur, de manière à exploiter la ventilation pour accélérer à la demande la diffusion des produits actifs en utilisant le système de réglage du débit d'air.

La présente demande de brevet constitue un perfectionnement de brevets du même inventeur et notamment :
- Le brevet français FR 2.764.806, qui concerne un diffuseur à plusieurs compartiments étanches contenant des perles parfumantes, se fixant de manière amovible sur toute grille de ventilation, diffusant régulièrement dans le temps des compositions aromatiques complexes, activé par l'ouverture successive de ses compartiments, chaque compartiment étant mis en service à la suite l'un de l'autre, on obtient une amélioration qualitative dans le temps en lieu et place d'un déclin inévitable avec tout autre procédé connu, aucun dispositif antérieur ne permettant d'additionner la performance résiduelle du compartiment précédent à celle du compartiment neuf suivant que l'on met en service.
- Le brevet français FR 2.875.137, qui décrit un dispositif de conditionnement de l'atmosphère par diffusion de substances aromatiques ou autres principes actifs volatils, constitué d'un flacon en verre associé à un diffuseur formé d'un bouchon entièrement en bois tourné comportant une tige plongeante également en bois immergée dans le principe actif contenu dans le flacon, le bouchon étant pourvu d'un pas de vis intérieur tourné dans la même pièce de bois permettant de le visser sur le col du flacon. Ce dispositif était à l'origine conçu pour être associé à un anneau ou un support permettant de l'intégrer en position verticale dans un système de ventilation. Ce support restait à concevoir, il est décrit dans la présente demande.

Aujourd'hui, les produits et procédés de parfumage d'ambiance, de bien-être autour de soi, et de confort de l'environnement doivent réunir au moins trois qualités indispensables :
1/ Un parfum d'ambiance et de confort doit être constitué d'un produit actif sain et sécurisé pour la santé humaine selon les dernières recherches en cosmétologie. La garantie élémentaire qu'un tel produit conçu pour être dispersé en aérosols dans l'air ambiant pour être respiré par l'utilisateur doit être de respecter les normes et usages de la grande parfumerie corporelle et de la cosmétologie, aussi bien que des enseignements de la phytothérapie si l'on utilise des huiles essentielles. Ce type de produit doit comporter uniquement des ingrédients acceptés dans les parfums corporels ou tout autre cosmétique pouvant entrer en contact avec la peau. Cet impératif concerne en premier lieu les principes actifs aromatiques, désodorisants ou autres, mais aussi tous les supports et solvants, ce qui implique d'éliminer tout élément soupçonné d'être cancérigène, perturbateurs endocriniens ou tous autres dangers aujourd'hui de mieux en mieux identifiés.
   Dans la présente invention les solvants seront de préférence remplacés par de l'alcool éthylique d'origine agricole, dans un dosage total approprié pour que le point éclair soit toujours supérieur à 21°C, et en tout état de cause d'un point éclair connu pour maîtriser le risque d'inflammabilité, afin de respecter les normes et règles actuelles concernant le stockage, l'emballage et l'étiquetage aussi bien que celles des transports terrestre, maritime ou aérien. Si l'on recherche un effet désodorisant, on utilisera de préférence l'acide undécylénique sous la forme d'un décylénate d'éthyle (C11) dont l'origine est une huile végétale : le ricin. On protégera avantageusement le produit actif par l'ajout de composé protecteur antioxydant, tel que I"'UVINUL A PLUS B" ® de la société BASF, assurant à la fois une protection des supports, de la composition active et de sa coloration. Lorsque l'on utilisera un colorant, il devra être alimentaire avec un dosage minimisé.
2/ La matière d'accueil du support solide ou le contenant du produit liquide devront également apporter la plus grande sécurité pour le stockage et la conservation prolongée des produits actifs. L'invention fera appel de préférence à l'un ou l'autre des 2 procédés suivants :
   - Pour faire une recharge solide, le stockage se fera dans le PEBAX ® (polyether block amide) utilisé en matière d'accueil dont les avantages ont été mis en évidence dans le brevet français n° FR 2.743.566. L'expérimentation portant aujourd'hui sur plus de 10 années a montré l'absence de dégradation dans le temps de l'ensemble complexe d'une composition de Parfumerie alcoolique malgré ses différents niveaux de volatilité.
   - Pour faire un parfum liquide de très haute concentration selon l'usage et des extraits et concentrés de parfum de haute qualité de la parfumerie traditionnelle, on effectuera le conditionnement dans un flacon verre, noble et recyclable associé à un diffuseur exclusivement constitué de matière naturelle 100 % biodégradable.
3/ Enfin, dans ces deux cas, le produit sera conçu pour respecter au mieux les règles connues destinées à préserver l'environnement écologique. Ainsi, un appareil diffuseur en matière plastique devra être chaque fois que possible mono matière ou fait d'une association de matière permettant d'être recyclable sans séparation des différents composants, ou à défaut détruit sans pollution dans des incinérateurs spéciaux pour récupérer l'énergie. La matière ne devra pas être dégradée par les produits actifs, et notamment les parfums et les huiles essentielles hautement concentrées ; avantageusement on utilisera un polypropylène ou similaire, mais, si l'on veut privilégier la transparence, toute autre matière résistant au parfum pourra être utilisée. Là encore, en règle générale on évitera toutes les associations de matières plastiques pouvant compliquer les opérations de recyclage.

Le dispositif selon la présente invention a pour objectif de tenir compte de ces contraintes. Il permet en effet le conditionnement de l'atmosphère par la diffusion de substances aromatiques et autres principes volatils respectant en permanence les plus récentes recherches et normes qualitatives connues en cosmétologie et, le cas échéant en phytothérapie. L'invention se présente donc comme un concept pertinent pour optimiser les performances des parfums à monter sur un système de ventilation dans le respect de la santé humaine, la plus grande sécurité à l'utilisation et le respect de l'environnement écologique.

On connaît par les documents brevet EP 1.627.648 (ZOBELE) et EP1.595.552 (SARA LEE), des diffuseurs d'aérosols comportant un support équipé d'un logement dans lequel une recharge à produit liquide peut être insérée, ladite recharge étant pourvue d'une mèche. Un chapeau mobile inséré dans le logement peut couvrir la mèche entièrement ou partiellement et capter le flux d'air d'un système d'aération pour le concentrer sur la mèche de la recharge. Dans ces documents, seul un modèle spécifique de recharge à produit liquide peut être inséré dans le logement prévu dans le support.

Les documents brevets US 4.523.870 (SPECTOR), US 6.102.660 (PAO-FENG) et WO 01/70284 (FIGIEL), divulguent également des diffuseurs d'aérosols comportant un support à l'intérieur duquel une recharge imprégnée de parfum est insérée. Le support et la recharge sont tous deux pourvus d'orifices agencés de manière à permettre à un flux d'air de traverser la recharge pour ressortir chargé de parfum. Dans ce document, seul un modèle spécifique de recharge peut être inséré dans le support.

Face à cet état des choses, l'objectif de l'invention est de proposer un diffuseur d'aérosols dans lequel peut être inséré non seulement des recharges à produit liquide du type décrit dans le brevet français FR 2.875.137 au nom de l'inventeur, mais également des recharges à produit solide.

### Divulgation de l'invention.

Il est constitué d'un support pouvant être associé à un système d'aération et formant un berceau-écrin polyvalent apte à recevoir plusieurs types de recharges à un ou plusieurs compartiments équipées d'un diffuseur et contenant une substance volatile liquide ou solide destinée à améliorer le confort de l'environnement, ledit support disposant de déflecteurs captant le flux d'air du système de ventilation pour le concentrer sur le diffuseur de la recharge.

Plus précisément, la solution technique proposée par l'invention est un diffuseur d'aérosols comportant un support destiné à être monté sur un aérateur d'un système de ventilation et des recharges interchangeables. Ce diffuseur est remarquable en ce que le support est formé d'une embase équipée d'un logement en forme de berceau-écrin polyvalent, ledit logement étant configuré pour recevoir successivement :
- le flacon d'une recharge à produit liquide contenant un concentré de parfum liquide, ledit flacon étant surmonté d'un bouchon diffuseur en bois,
- le récipient d'une recharge à produit solide contenant des granulés parfumés, ledit récipient étant surmonté d'un diffuseur,
   Ce diffuseur est en outre remarquable en ce que le logement est surmonté d'un déflecteur d'air disposé en vis-à-vis du bouchon diffuseur en bois de la recharge à produit liquide ou du diffuseur de la recharge à produit solide.

Le logement en forme de berceau-écrin est avantageusement délimité par deux parois latérales dont les bords sont reliés par une paroi de fond et dont les extrémités inférieures sont reliées par une paroi support sur laquelle reposent les recharges, la face avant dudit logement étant ouverte de façon à permettre l'insertion desdites recharges dans ledit logement.

Les recharges sont préférentiellement maintenues en position dans le logement par l'intermédiaire d'un élément de maintien comportant deux éléments latéraux de fixation par encliquetage sur l'embase se terminant par deux pattes aboutissant à l'arrière de ladite embase et devant être poussées exactement en même temps pour libérer lesdites recharges, de façon à constituer une sécurité enfant.

Un clip orientable peut être monté à l'arrière de l'embase, ledit clip étant constitué d'une pince articulée avec rotule.

Préférentiellement, l'embase, l'élément de maintien de la recharge sur ladite embase et le clip orientable sont réalisés dans la même matière.

Une recharge à produit solide destinée à être utilisée dans le dispositif d'aérosols conforme à l'invention, est avantageusement constituée :
- d'un récipient à trois compartiments séparés par des cloisons verticales et apte à contenir des perles parfumantes, le fond dudit récipient comportant une entrée d'air formée d'orifices,
- d'un couvercle fermant le récipient à sa partie supérieure et pourvu de perforations calculées pour que les perles ne puissent pas sortir seules, même après désorption de leur produit actif et réduction proportionnelle de leur volume,
- d'un obturateur rotatif à axe vertical plaqué sur le couvercle et comportant une ouverture déterminée pour permettre, à l'aide d'un levier de fermer le récipient, ou de mettre en fonction un, deux ou trois compartiments successivement ou simultanément pour additionner leurs performances, et
- d'un élément diffuseur surmontant l'obturateur et agencé pour diriger l'air chargé de produit actif horizontalement vers l'avant.

Pour faciliter la conception, le diffuseur, le récipient et le couvercle peuvent être moulés en une seule pièce à plat , sous la forme d'un boîtier en charnière arrière et emboîtée de façon étanche à l'avant, conçu de façon à intégrer dans la même pièce les cloisons verticales et le diffuseur, seul l'obturateur équipé du levier, étant moulé séparément.

Dans cette recharge à produit solide, les perles parfumantes sont préférentiellement formées de granulés de résine polymère du type polyéther-ester amide imprégnés de compositions parfumées complexes ou de tout autre produit actif destiné à l'amélioration du confort de l'environnement.

Le récipient de la recharge à produit solide peut comporter, en face avant, des échelles de mesure bien visibles par l'utilisateur pour montrer le niveau de perles en cours d'utilisation.

L'élément diffuseur surmontant l'obturateur de la recharge à produit solide, peut être avantageusement agencé pour utiliser l'effet d'aspiration Venturi grâce à un obturateur rotatif comportant un conduit évasé de passage de l'air de façon à permettre une fermeture étanche totale du récipient en couvrant les perforations du couvercle supérieur et en supprimant les ouvertures du fond dudit récipient.

Une recharge à produit liquide destinée à être utilisée dans le dispositif d'aérosols conforme à l'invention, est préférentiellement constituée d'un flacon contenant un concentré de parfum liquide et fermé par un bouchon diffuseur en bois équipé d'une mèche également en bois enfoncée en force et sans colle et plongeant dans le concentré liquide.

Cette recharge à produit liquide peut comporter une coupelle en résine polyether bloc amide PEEA disposée entre le bouchon diffuseur et le flacon et agencée pour récupérer les suintements extérieurs excessifs, les stocker par absorption et les rediffuser ensuite avec régularité dans le temps. Une lèvre remontante est avantageusement organisée à l'intérieur de la coupelle pour venir s'insérer à l'intérieur du bas du bouchon diffuseur de façon à assurer une double sécurité d'étanchéité contre les suintements.

Le bouchon diffuseur de la recharge à produit liquide pourra comporter un pas de vis sculpté dans la masse calculé très exactement pour être toujours jointif entre le dessus du col du flacon et le fond dudit bouchon de façon à assurer, même après dilatation, une étanchéité suffisante, ladite étanchéité étant améliorée au moyen d'un joint souple percé disposé au fond du bouchon et traversé par la mèche en bois.

Dans une variante de réalisation, la mèche est configurée de manière à laisser un espace au niveau du col du flacon et un espace au niveau de son logement dans le bouchon, ces espaces permettant le retour du liquide par ruissellement à l'intérieur dudit flacon.

Le flacon de la recharge à produit liquide est avantageusement sécurisé par un bouchon de transport étanche destiné à être retiré avant l'utilisation, et remplacé par le bouchon diffuseur en bois déjà équipé de sa mèche. Dans une variante de réalisation, la recharge à produit liquide est constituée d'un flacon contenant un concentré de parfum liquide, ledit flacon étant sécurisé par :
- un obturateur cylindrique de type topette inséré dans le col dudit flacon,
- un bouchon diffuseur en bois dépourvu de mèche, fixé sur le col dudit flacon et recouvrant l'obturateur cylindrique,
ledit obturateur cylindrique étant destiné à être retiré avant utilisation et remplacé par une mèche en bois montée ultérieurement sur ledit bouchon et enfoncée en force et sans colle et plongeant dans le concentré liquide.

Un autre aspect de l'invention est un support de présentation pour le diffuseur d'aérosols conforme aux caractéristiques précédentes, ledit support consistant en un emballage écologique mono-matière entièrement réalisé dans une simple feuille découpée de carton pliée non collée, enveloppant le diffuseur (avec les deux recharges, ou soit l'une soit l'autre, ou aucune des deux), ledit diffuseur étant fixé sur ledit support par une double attache élastique posée en une seule opération par une double aiguille et assurant à la fois l'assemblage de l'emballage, le maintien du diffuseur et une inviolabilité correcte.

Encore un autre aspect de l'invention est un ensemble comportant un aérateur de système de ventilation et un diffuseur d'aérosols conforme aux caractéristiques précédentes, ledit diffuseur étant agencé sur ledit aérateur de sorte que le déflecteur d'air dudit dispositif capte le flux d'air dudit système de ventilation pour le concentrer sur le bouchon diffuseur en bois de la recharge à produit liquide ou du diffuseur de la recharge à produit solide.

### Description des figures.

Sur les dessins annexés, donnés à titre d'exemples non limitatifs de formes de réalisation de l'objet de l'invention :
- la figure 1 représente, vu de l'avant en perspective, le support et son élément de maintien de la recharge séparés,
- la figure 2 montre dans les mêmes conditions le support vu de l'arrière,
- la figure 3 est une vue éclatée en perspective d'une recharge à trois compartiments pour substance volatile solide,
- la figure 4 montre le récipient de la recharge de la figure précédente vu de dessous,
- la figure 5 représente le support et son élément de maintien de la recharge assemblés,
- la figure 6 montre la recharge de la figure 3 installée dans le support,
- la figure 7 est une vue éclatée en perspective d'une recharge à bouchon diffuseur en bois pour substance volatile liquide,
- la figure 8 représente la recharge de la figure précédente avec ses constituants assemblés,
- la figure 9 montre la recharge de la figure 8 installée dans le support,
- la figure 10 est une vue éclatée en perspective d'une variante de recharge à trois compartiments pour substance volatile solide utilisant l'effet Venturi pour diffuser la substance volatile,
- et la figure 11 montre le récipient de la recharge de la figure 10 vu de dessous.

### Modes de réalisation de l'invention.

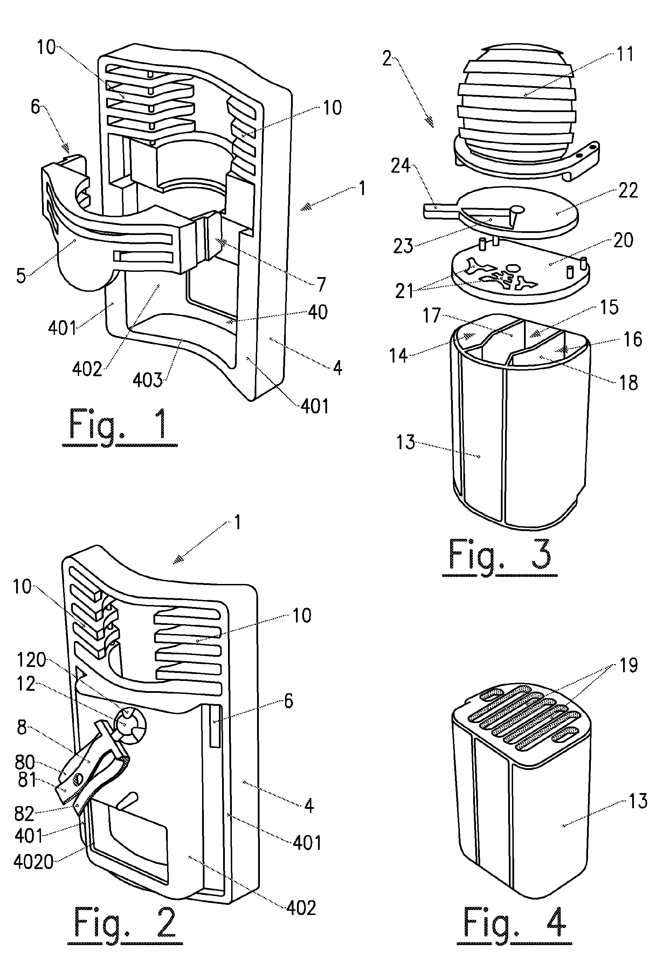

Le dispositif diffuseur, figures 1 à 11, est constitué d'un support 1 réalisé de préférence en matière synthétique moulée tel que le polypropylène ou similaire et destiné à recevoir une recharge 2, 3 interchangeable de substance volatile, le support 1 étant formé d'une embase 4 et d'un élément de maintien 5 de la recharge comportant deux éléments latéraux 6, 7 de fixation par encliquetage sur l'embase 4.

Plus précisément, et en se rapportant aux figures annexées, le diffuseur objet de l'invention est remarquable en ce que le support 1 est formé d'une embase 4 équipée d'un logement 40 en forme de berceau-écrin polyvalent, ledit logement étant configuré pour recevoir successivement :
- le flacon 25 d'une recharge 3 à produit liquide contenant un concentré de parfum liquide, ledit flacon étant surmonté d'un bouchon diffuseur en bois 9,
- le récipient 13 d'une recharge 2 à produit solide contenant des granulés parfumés, ledit récipient étant surmonté d'un diffuseur 11.

Le support 1 est destiné à être monté sur l'aérateur d'un système de ventilation. À titre d'exemple, le système de diffusion peut être fixé de façon amovible par l'utilisateur sur une grille d'aérateur grâce à un clip 8 orientable monté à l'arrière de l'embase 4 (figure 2). Ladite embase, l'élément de maintien 5 de la recharge et le clip 8 orientable étant avantageusement réalisés dans la même matière. Le dispositif peut également être intégré dans un système d'aération. Il peut en particulier être associé au système interne de ventilation/climatisation d'une automobile directement en première monte c'est-à-dire dès la conception des circuits et aérateurs par le constructeur automobile. S'il s'agit d'une utilisation dans un bureau, le dispositif de diffusion de la présente invention pourra avantageusement être clipsé, fixé ou intégré à un appareil de bureautique, domestique, ou autre, muni d'un système de ventilation, qu'il s'agisse d'un ordinateur, d'une imprimante, d'un télécopieur ou tout autre matériel soufflant un air ventilé vers l'extérieur.

Nous retiendrons ici à titre d'exemple non limitatif un concept de diffuseur polyvalent pour deux types de recharges 2, 3 ; ces recharges venant toutes se loger dans le même support 1 berceau-écrin universel d'aspect esthétique et valorisant qui trouvera sa place sur toute grille d'aération voiture, maison, collectivité ou autre.

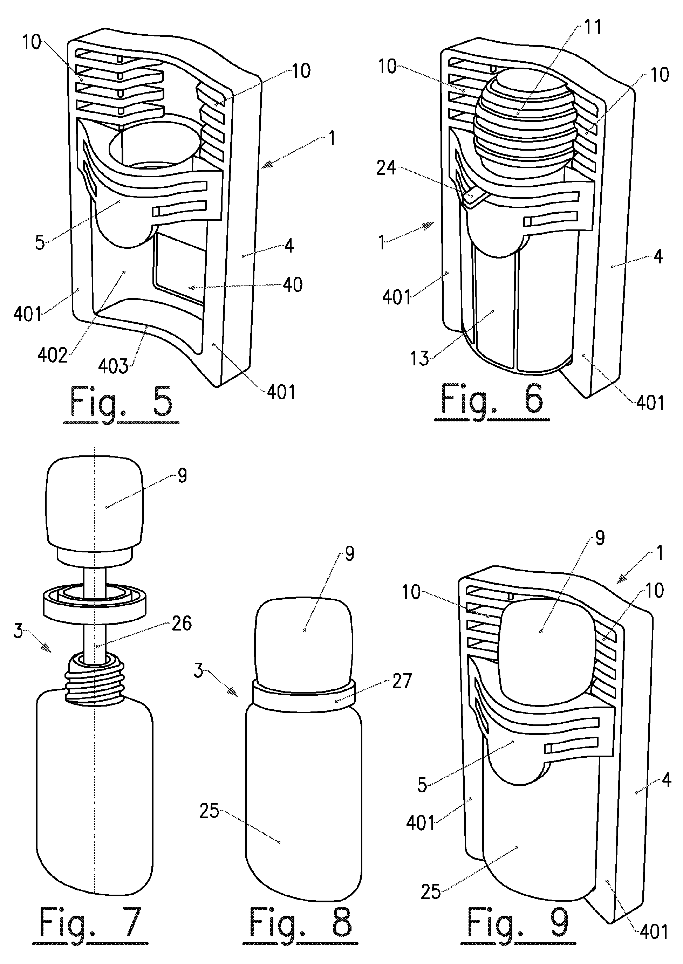

Le support 1 sera réalisé dans toutes formes, matières et dimensions, pour servir de support aux recharges à contenu solide 2 (figures 3, 4, 6, 10, 11) ou à contenu liquide 3 à bouchon diffuseur 9 en bois (figures 7 à 9). Il pourra avantageusement et sans surcoût du moulage comporter des reliefs décoratifs. De plus, l'écrin universel disposera de déflecteurs 10 captant le flux d'air venant du système d'aération pour le concentrer au centre directement sur le bouchon bois 9 ou dans l'élément diffuseur 11 prévu à la partie supérieure de la recharge 2 à contenu solide, l'objectif étant l'optimisation du flux d'air pour les deux types de recharges de parfum.

En se rapportant aux figures 1, 2 et 5, le logement 40 en forme de berceau-écrin est délimité par deux parois latérales 401 dont les bords sont reliés par une paroi de fond 402 et dont les extrémités inférieures sont reliées par une paroi support 403 sur lequel repose les recharges 2, 3. Le logement 40 est ouvert sur sa face avant de façon à permettre l'insertion des recharges 2, 3 dans ledit logement. L'élément de maintien 5 se fixe sur la face avant du logement 40, sous le déflecteur 10 du support 1, de manière à venir enserrer les recharges 2, 3 au niveau de la partie supérieure du flacon 25 ou du récipient 13, respectivement sous le bouchon bois 9 ou sous l'élément diffuseur 11. La paroi de fond 402 sera avantageusement pourvue d'une ouverture 4020 permettant de visualiser un éventuelle code-barre disposé à l'arrière du flacon 25 ou du récipient 13, des recharges 2, 3.

Dans le cas du support 1 amovible, le clip 8 arrière aura la forme d'une pince articulée avec rotule 12 capable de solidement tenir en place le diffuseur et de se fixer également fermement par serrage ou clipsage sur le plus grand nombre possible de volets ou d'ailettes de grille d'aérateur. Cette pince pourra être avantageusement interchangeable, de façon à permettre, au besoin, par simple changement de pince de s'adapter à tout type de grille ou d'appareils. Elle devra avoir les dimensions requises pour ne pas gêner le fonctionnement de l'aérateur ni l'accès aux systèmes d'ouverture et de fermeture. En se rapportant à la figure 2, la pince est fixée sur la rotule 12, elle-même montée dans un support sphérique 120 situé à l'arrière de l'embase 4 et solidaire de cette dernière, permettant de l'orienter dans le sens désiré. Cette disposition permet, grâce à un simple changement de pince, d'utiliser le diffuseur dans toutes les situations et dans toutes les orientations. Cette pince pourra notamment être constituée de trois ailettes 80, 81, 82 dont l'une est opposée aux deux autres (figure 2). En pratique, l'ailette centrale 81 pénètre au milieu des deux autres 80, 82 de manière à former la pince. Pour des considérations de recyclage, la pince 9 est avantageusement réalisée dans la même matière que le boîtier 1, préférentiellement en polypropylène car résistant aux huiles essentielles, pour obtenir un ensemble mono-matière. De ce fait, il est avantageux de prévoir sur chaque ailette 80, 81, 82 des renforts permettant de compenser la faible mémoire de forme de cette matière. Ce type de pince permet de s'adapter à toutes les situations verticales ou horizontales inclinées ou non avec des ailettes minces ou épaisses, c'est à dire concrètement aussi bien sur un aérateur de voiture, que sur une sortie d'air de climatiseur dans un local, ou sur la ventilation d'un appareil de bureautique.

La conception de l'élément de maintien 5 des différentes recharges lui permettra d'être utilisable de façon compréhensive par le consommateur qui pourra l'enlever et le remettre aisément en jouant ainsi sur l'interchangeabilité, donc l'économie-écologie d'un diffuseur permanent dont on ne changera que la recharge. De plus, les deux éléments latéraux 6, 7 de fixation par encliquetage se terminent par deux pattes aboutissant à l'arrière de l'embase 4 et devant être poussées exactement en même temps pour libérer une recharge, 2, 3, ce qui constitue une sécurité enfant indispensable.

La recharge 2 à produit solide (figures 3 et 4) est avantageusement constituée d'un récipient 13 à trois compartiments 14, 15, 16 séparés par des cloisons verticales 17, 18 et contenant de préférence des perles parfumantes formées de granulés de résine polymère du type polyéther-ester amide imprégnés de compositions parfumées complexes ou de tout autre produit actif destiné à l'amélioration du confort de l'environnement. On peut toutefois prévoire d'utiliser un nombre supérieur ou inférieur de compartiments et de cloisons. La dimension du récipient 13 permettra d'accueillir entre 5 et 30 g de perles parfumantes, de préférence 10 à 15 g pour 2 mois d'utilisation dans l'automobile.
Les granulés seront avantageusement du type AROMAPERL® constitués de la résine polyether bloc amide (PEEA) décrite dans le brevet N° FR 2.743.566 (nom commercial "PEBAX")
Le fond du récipient 13 comporte une entrée d'air formée d'orifices 19 adaptés pour laisser passer une partie du flux d'air et dont la surface sera de préférence comprise entre 20 à 50 mm2 de façon à réguler correctement le débit d'air à travers les perles parfumantes (figure 4).
Le récipient 13 est fermé à sa partie supérieure par un couvercle 20 pourvu de perforations 21 calculées pour que les perles ne puissent pas sortir seules, même après désorption de leur produit actif et réduction proportionnelle de leur volume.
Le couvercle 20 est surmonté d'un obturateur 22 rotatif à axe vertical comportant une ouverture 23 déterminée pour permettre, grâce à un levier 24 passant à travers l'élément de maintien 5, de passer successivement aux trois phases d'utilisation, à la volonté de l'utilisateur, tout en permettant aussi de fermer et d'ouvrir progressivement les compartiments. L'obturateur 22 permet une position fermée puis trois positions ouvrant de manière cumulative les trois compartiments 14, 15, 16 du récipient 13.
Ainsi, on pourra effectivement bénéficier de l'amélioration qualitative dans le temps en mettant en fonction le compartiment 14 seul, puis les compartiments 14 et 15 ensemble, puis les trois compartiments ensemble.
L'obturateur 22 est surmonté d'un élément diffuseur 11 agencé pour diriger l'air chargé de produit actif horizontalement vers l'avant.
On pourra faire apparaître en face avant du récipient 13 des échelles de mesure bien visibles par l'utilisateur pour montrer le niveau de perles en cours d'utilisation et faire en sorte que lorsque leur volume sera réduit après la diffusion des actifs, le niveau visible baisse jusqu'à montrer un espace vide repéré qui indiquera qu'il est opportun de passer au compartiment suivant, ou de changer de container recharge.

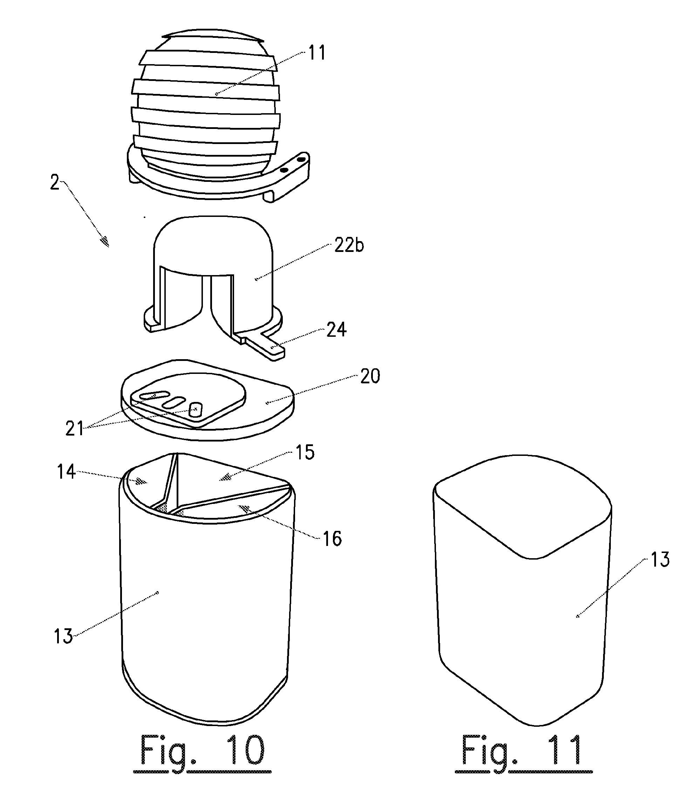

Les figures 10 et 11 illustrent une variante de recharge 2 à produit solide destinée à obtenir une fermeture étanche totale du récipient 13 en conservant les systèmes d'obturations supérieurs et en supprimant les ouvertures 19 du fond du récipient 13.
Dans ce cas, l'élément diffuseur 11 surmontant l'obturateur sera agencé pour utiliser l'effet d'aspiration Venturi grâce à un obturateur rotatif 22b comportant un conduit évasé de passage de l'air, de façon à permettre une fermeture étanche totale du récipient 13 et une ouverture progressive et cumulative dudit récipient.

L'effet Venturi est un effet d'aspiration par un fluide en mouvement. En effet, si la pression diminue, alors il y a aspiration. Dans un tuyau avec débit constant, lorsque la section de passage est successivement diminuée puis augmentée, la vitesse croît et la pression décroît, ce qui permet de créer un effet d'aspiration dans la zone de plus faible section du tuyau.
Cette solution, particulièrement bien adaptée à l'intégration dans un circuit de ventilation, notamment l'automobile sera préférée lorsque le constructeur automobile ou le fabricant d'appareils électroniques ventilé, par exemple un fax, aura la possibilité d'aménager le flux d'air pour produire une aspiration suffisante des parfums ou matières volatiles et l'extraire d'un ou plusieurs compartiments pouvant être mis en service séparément, successivement ou ensemble.

Un autre mode de réalisation de la recharge 2 peut être de mouler en une seule pièce à plat : le diffuseur 11, le récipient 13 et le couvercle 20, sous la forme d'un boîtier en charnière arrière et emboîtée de façon étanche à l'avant, conçu de façon à intégrer dans la même pièce les cloisons internes verticales 17 et 18 et le diffuseur 11. Seul l'obturateur 22, équipé du levier 24, est moulé séparément et ajusté dans des logements appropriés.

La recharge à produit liquide 3 (figures 7 et 8) est avantageusement constituée d'un flacon 25 à bouchon diffuseur 9 en bois contenant un concentré de parfum liquide. Le bouchon est équipé d'une mèche 26 en bois et est sécurisé par un bouchon de transport étanche. Avant l'utilisation, le bouchon de transport est retiré, puis est remplacé par le bouchon diffuseur 9 doté de sa mèche 26 en bois qui plonge dans le parfum liquide. Dans une variante de réalisation non représentée, le flacon de la recharge à produit liquide est sécurisé par :
- un obturateur cylindrique de type topette inséré dans le col dudit flacon,
- le bouchon diffuseur en bois dépourvu de sa mèche, fixé sur le col du flacon et recouvrant l'obturateur cylindrique. La mèche est livrée séparément et montée ultérieurement sur le bouchon par l'utilisateur final.
   En service, le bouchon en bois 9 est idéalement placé dans le flux d'air concentré par les déflecteurs 10 du support 1.
   Les suintements excessifs extérieurs sont récupérés par une coupelle 27 en PEBAX® disposée entre le bouchon et le flacon 25 et apte à les stocker par absorption et les rediffuser ensuite avec régularité dans le temps. Par ailleurs, une lèvre remontante est organisée à l'intérieur de la coupelle pour venir de préférence par un emboîtement conique, s'insérer à l'intérieur du bas du bouchon bois assurant ainsi une double sécurité d'étanchéité pour les suintements provenant du pas de vis à l'intérieur du bouchon.
   L'invention objet du brevet français FR 2.875.137 décrivant un flacon contenant un parfum de très haute concentration associé à un bouchon diffuseur en bois pur produit artisanal constitue un inégalable évaporateur au naturel, très supérieur aux associations de matières plastiques ce qui prouve que la nature fait souvent très bien les choses et qu'il est sage de s'en inspirer.
   Trois années de test de parfumage ont en effet montré la remarquable adéquation entre les concentrés de parfums traditionnels très riches en huiles essentielles et l'évaporation régulière qu'il est possible d'obtenir avec un bois judicieusement sélectionné, de préférence du hêtre, avec une tige plongeante également en bois d'un diamètre bien adapté.
   La conception du bouchon diffuseur 9 avec un pas de vis sculpté dans la masse, équipé d'une mèche 26 en bois enfoncée en force et sans colle, le pas de vis étant très exactement calculé pour être toujours jointif entre le dessus du col du flacon 25 et le fond du bouchon assureront surtout après dilatation une étanchéité suffisante, cette étanchéité étant par ailleurs améliorée par un joint souple percé au fond du bouchon, traversé par la mèche tige en bois. Ce joint souple peut être soit un joint plat soit un joint torique.
   Une des principales difficultés rencontrées pour les diffuseurs de parfum liquide en flacon avec mèche généralement en polyester, est l'excès d'évaporation surtout lorsque le diffuseur est placé sur l'aérateur d'un véhicule, derrière le pare brise où les températures au soleil peuvent atteindre 60 °C à 65 °C. Il en est de même lorsque la ventilation du chauffage se met en fonction avec une température extérieure basse (par exemple -10°C), l'air pulsé peut également dépasser 60 °C à 65 °C.
   Dans ce cas, la pression interne du liquide volatil est telle que l'évaporation très excessive doit être récupérée dans une sorte d'entonnoir pour retourner dans le flacon. Il s'ensuit que le flacon ne peut jamais être étanche, qu'il doit être absolument en position verticale, que la moindre déviation fait ruisseler le liquide à l'extérieur et provoque inévitablement la destruction des matières plastiques, bois, cuir, etc, souvent très coûteuses.
   Le bouchon bois 9 bien vissé, de plus jointé, ne peut laisser diffuser un excès de liquide concentré parfumant qui ne soit pas évaporé, mais par sécurité ultime, l'invention comporte en plus la coupelle 27 de récupération moulée dans la même matière d'accueil que les granulés AROMAPERL® et constituant ainsi une deuxième étanchéité de sécurité.
   On peut donc dire que la recharge flacon + bouchon bois avec coupelle de récupération apportera une sécurité tableau de bord par rétention des suintements et régulation de l'évaporation qui n'existent pas à ce jour.
   Pour les situations extrêmes de hautes températures (supérieures à environ 60°C) ou dans tous les autres cas où il est utile de récupérer les suintements du liquide, la mèche 26 peut être configurée de manière à laisser un espace au niveau du col du flacon 25 et un espace au niveau de son logement dans le bouchon 9, ces espaces permettant le retour du liquide par ruissellement à l'intérieur dudit flacon. Cet aménagement peut consister à utiliser non pas une mèche cylindrique, mais une mèche ½ rond ou par exemple ¾ de rond, ou encore en réalisant des rainures longitudinales sur ladite mèche, de sorte que le liquide en excès sous pression puisse revenir par ruissellement à l'intérieur du flacon 25.
   La présentation du support 1 et des recharges 2, 3 de parfum liquide ou solide sur les lieux de vente pourra avantageusement être effectuée grâce à un support de présentation consistant en un emballage écologique mono-matière entièrement réalisé dans une simple feuille découpée de carton pliée non collée, enveloppant le diffuseur (avec les deux recharges, ou soit l'une soit l'autre, ou aucune des deux), ledit diffuseur étant visible à l'intérieur dudit support par une fenêtre, le diffuseur étant fixé sur la partie arrière dudit support par une double attache élastique de type « VSN de DENISSON® » posée en une seule opération par une double aiguille et assurant à la fois l'assemblage de l'emballage, le maintien du diffuseur et une inviolabilité correcte.
   Le positionnement des divers éléments constitutifs donne à l'objet de l'invention un maximum d'effets utiles qui n'avaient pas été, à ce jour, obtenus par des dispositifs similaires.

### Documents connus de l'état de la technique.

- FR 2.764.806 (JEAN)
- FR 2.875.137 (JEAN)
- EP 1.627.648 (ZOBELE)
- EP1.595.552 (SARA LEE)
- US 4.523.870 (SPECTOR)
- US 6.102.660 (PAO-FENG)
- WO 01/70284 (FIGIEL).

## Revendications

1. Diffuseur d'aérosols comportant un support (1) destiné à être monté sur un aérateur d'un système de ventilation et des recharges (2, 3) interchangeables,
**se caractérisant par le fait que** ledit support est formé d'une embase (4) équipée d'un logement (40) en forme de berceau-écrin polyvalent, ledit logement étant configuré pour recevoir successivement :
- le flacon (25) d'une recharge (2) à produit liquide contenant un concentré de parfum liquide, ledit flacon étant surmonté d'un bouchon diffuseur en bois (9),
- le récipient (13) d'une recharge (3) à produit solide contenant des granulés parfumés, ledit récipient étant surmonté d'un diffuseur (11),
et **par le fait que** le logement (40) est surmonté d'un déflecteur d'air (10) disposé en vis-à-vis du bouchon diffuseur en bois (9) de la recharge (2) à produit liquide ou du diffuseur (11) de la recharge (3) à produit solide.

2. Diffuseur d'aérosols selon la revendication 1, **se caractérisant par le fait que** le logement (40) en forme de berceau-écrin est délimité par deux parois latérales (401) dont les bords sont reliés par une paroi de fond (402) et dont les extrémités inférieures sont reliées par une paroi support (403) sur laquelle reposent les recharges (2, 3), la face avant dudit logement étant ouverte de façon à permettre l'insertion desdites recharges dans ledit logement.

3. Diffuseur d'aérosols selon l'une quelconque des revendications précédentes, **se caractérisant par le fait que** les recharges (2, 3) sont maintenues en position dans le logement par l'intermédiaire d'un élément de maintien (5) comportant deux éléments latéraux (6, 7) de fixation par encliquetage sur l'embase (4) se terminant par deux pattes aboutissant à l'arrière de ladite embase et devant être poussées exactement en même temps pour libérer lesdites recharges, de façon à constituer une sécurité enfant.

4. Diffuseur d'aérosols selon l'une quelconque des revendications précédentes, **se caractérisant par le fait qu'**il comporte un clip (8) orientable monté à l'arrière de l'embase (4) et constitué d'une pince articulée avec rotule (12).

5. Recharge (2) à produit solide destinée à être utilisée dans le dispositif d'aérosols selon l'une quelconque des revendications précédentes, ladite recharge étant constituée :
- d'un récipient (13) à trois compartiments (14, 15, 16) séparés par des cloisons verticales (17, 18) et apte à contenir des perles parfumantes, le fond dudit récipient comportant une entrée d'air formée d'orifices (19),
- d'un couvercle (20) fermant le récipient (13) à sa partie supérieure et pourvu de perforations (21) calculées pour que les perles ne puissent pas sortir seules, même après désorption de leur produit actif et réduction proportionnelle de leur volume,
- d'un obturateur (22) rotatif à axe vertical plaqué sur le couvercle (20) et comportant une ouverture (23) déterminée pour permettre, à l'aide d'un levier (24) de fermer le récipient (13), ou de mettre en fonction un, deux ou trois compartiments (14, 15, 16) successivement ou simultanément pour additionner leurs performances, et
- d'un élément diffuseur (11) surmontant l'obturateur (22) et agencé pour diriger l'air chargé de produit actif horizontalement vers l'avant.

6. Recharge à produit solide selon la revendication 5, dans lequel le diffuseur (11), le récipient (13) et le couvercle (20) sont moulée en une seule pièce à plat, sous la forme d'un boîtier en charnière arrière et emboîtée de façon étanche à l'avant, conçu de façon à intégrer dans la même pièce les cloisons verticales (17, 18) et le diffuseur (11), seul l'obturateur (22) équipé du levier (24), étant moulé séparément.

7. Recharge (2) à produit solide destinée à être utilisée dans le dispositif d'aérosols selon l'une quelconque des revendications 1 à 4, ladite recharge étant constituée :
- d'un récipient (13) à trois compartiments (14, 15, 16) séparés par des cloisons verticales (17, 18) et apte à contenir des perles parfumantes, le fond dudit récipient étant dépourvu d'orifices,
- d'un couvercle (20) fermant le récipient (13) à sa partie supérieure et pourvu de perforations (21) calculées pour que les perles ne puissent pas sortir seules, même après désorption de leur produit actif et réduction proportionnelle de leur volume,
- d'un obturateur (22) rotatif à axe vertical plaqué sur le couvercle (20) et comportant une ouverture (23) déterminée pour permettre, à l'aide d'un levier (24) de fermer le récipient (13), ou de mettre en fonction un, deux ou trois compartiments (14, 15, 16) successivement ou simultanément pour additionner leurs performances, et
- d'un élément diffuseur (11) surmontant l'obturateur (22) et agencé pour diriger l'air chargé de produit actif horizontalement vers l'avant, ledit élément diffuseur étant agencé pour utiliser l'effet d'aspiration Venturi grâce à un obturateur rotatif (22b) comportant un conduit évasé de passage de l'air de façon à permettre une fermeture étanche totale du récipient (13) et une ouverture progressive et cumulative dudit récipient.

8. Recharge à produit solide selon l'une des revendications 5 à 7, **se caractérisant par le fait que** les perles parfumantes sont formées de granulés de résine polymère du type polyéther-ester amide imprégnés de compositions parfumées complexes ou de tout autre produit actif destiné à l'amélioration du confort de l'environnement.

9. Recharge à produit solide selon l'une des revendications 7 à 8, **se caractérisant par le fait que** le récipient (13) comporte, en face avant, des échelles de mesure bien visibles par l'utilisateur pour montrer le niveau de perles en cours d'utilisation

10. Recharge (3) à produit liquide destinée à être utilisée dans le dispositif d'aérosols selon l'une quelconque des revendications 1 à 4, ladite recharge étant constituée d'un flacon (25) contenant un concentré de parfum liquide et fermé par un bouchon diffuseur (9) en bois équipé d'une mèche (26) également en bois enfoncée en force et sans colle et plongeant dans le concentré liquide.

11. Recharge (3) à produit liquide destinée à être utilisée dans le dispositif d'aérosols selon l'une quelconque des revendications 1 à 4, ladite recharge étant constituée d'un flacon (25) contenant un concentré de parfum liquide, ledit flacon étant sécurisé par :
- un obturateur cylindrique de type topette inséré dans le col dudit flacon
- un bouchon diffuseur en bois dépourvu de mèche, fixé sur le col dudit flacon et recouvrant l'obturateur cylindrique,
ledit obturateur cylindrique étant destiné à être retiré avant utilisation et remplacé par une mèche (26) en bois montée ultérieurement sur ledit bouchon et enfoncée en force et sans colle et plongeant dans le concentré liquide.

12. Recharge à produit liquide selon l'une des revendications 10 ou 11, **se caractérisant par le fait qu'**elle comporte une coupelle (27) en résine polyether bloc amide PEEA disposée entre le bouchon diffuseur (9) et le flacon (25) et agencée pour récupérer les suintements extérieurs excessifs, les stocker par absorption et les rediffuser ensuite avec régularité dans le temps.

13. Recharge à produit liquide selon l'une des revendications 10 à 12, **se caractérisant par le fait que** le bouchon diffuseur (9) comporte un pas de vis sculpté dans la masse calculé très exactement pour être toujours jointif entre le dessus du col du flacon (25) et le fond dudit bouchon de façon à assurer, même après dilatation, une étanchéité suffisante, ladite étanchéité étant améliorée au moyen d'un joint souple percé disposé au fond du bouchon et traversé par la mèche (26) en bois.

14. Recharge à produit liquide selon l'une des revendications 10 ou 11, **se caractérisant par le fait que** la mèche (26) est configurée de manière à laisser un espace au niveau du col du flacon (25) et un espace au niveau de son logement dans le bouchon (9), ces espaces permettant le retour du liquide par ruissellement à l'intérieur dudit flacon.

15. Ensemble comportant un support de présentation et le diffuseur d'aérosols conformes aux revendications 1 à 4, ledit support de présentation consistant en un emballage écologique mono-matière entièrement réalisé dans une simple feuille découpée de carton pliée non collée, enveloppant le diffuseur, ce dernier étant fixé sur ledit support par une double attache élastique posée en une seule opération par une double aiguille et assurant à la fois l'assemblage de l'emballage et le maintien dudit diffuseur.

16. Ensemble comportant un aérateur de système de ventilation et un diffuseur d'aérosols conforme à l'une des revendications 1 à 4, ledit diffuseur étant agencé sur ledit aérateur de sorte que le déflecteur d'air (10) dudit dispositif capte le flux d'air dudit système de ventilation pour le concentrer sur le bouchon diffuseur en bois (9) de la recharge (2) à produit liquide ou du diffuseur (11) de la recharge (3) à produit solide.
